# DEMANDE DE BREVET EUROPEEN

(11) **EP 1 186 660 A2**
(43) Date de publication de la demande: **13.03.2002**
(21) Numéro de dépôt: 01126393.6
(22) Date de dépôt: 17.06.1985
(51) Int. Cl.: C12N 15/10

(54) **Procédé d'obtension d'ADN, ARN, peptides, polypeptides ou protéines, par une technique de recombination d'ADN**

(30) Priorité: 30.03.1985 CH 137985
(62) Demande divisionnaire de: 93116225.9
(71) Demandeur: KAUFFMAN, Stuart A., Santa Fe, NM 87501 (US)
(72) Inventeur: Kauffmann, Stuart Alan, Santa Fe, New Mexico 87501 (US); Ballivet, Marc, 1208 Geneve (CH)
(74) Mandataire: Lawrence, John

(57) **Abrégé**

Pour l'obtention d'un ensemble de peptides, polypeptides ou protéines capable de catalyser une séquence de réactions chimiques conduisant à la formation d'au moins un composé désiré, on produit une bibliothèque de gènes au moins partiellement constitués de polynucléotides synthétiques stochastiques, on amplifie ces gènes et on effectue un criblage de façon à identifier une pluralité de gènes capables d'être exprimés sous forme d'un ensemble de peptides, polypeptides ou protéines ayant l'aptitude catalytique désirée, définie ci-dessus. On utilise ensuite l'information génétique ainsi obtenue pour produire en ensemble de peptides, polypeptides ou protéines ayant ladite aptitude catalytique. On peut finalment utiliser cet ensemble pour la production d'un composé chimique, éventuellement nouveau, ayant une propriété donnée.

## Description

La présente invention a pour objet un procédé d'obtention d'ADN, ARN, peptides, polypeptides ou protéines, au moyen de cellules-hôtes modifiées contenant des gênes capables d'exprimer ces ARN, peptides, polypeptides ou protéines, c'est-à-dire en utilisant une technique de recombinaison d'ADN.

L'invention vise notamment à produire des gênes ou fragments de gènes stochastiques, de façon à permettre l'obtention simultanée, après transcription et traduction de ces gènes, d'un très grand nombre (de l'ordre de dix mille au moins) de protéines complètement nouvelles ou hybrides de protéines connues, en présence de cellules-hôtes (souches bactériennes ou eucaryotes) contenant les gènes respectivement capables d'exprimer ces protéines et d'effectuer ensuite une sélection ou criblage parmi lesdites souches, en vue de déterminer celles qui produisent des protéines présentant des propriétés désirées, par exemple des propriétés structurelles enzymatiques, catalytiques, antigéniques, pharmacologiques, ou des propriétés de ligand, et plus généralement, des propriétés chimiques, biochimiques, biologiques, etc.

L'invention a également pour but de permettre l'obtention de séquences d'ADN ou d'ARN présentant des propriétés utilisables, notamment des propriétés chimiques, biochimiques ou biologiques.

On comprend donc que l'invention est susceptible de recevoir de très nombreuses applications, dans des domaines très variés de la science, de l'industrie et de la médecine.

Le procédé de production de peptides ou polypeptides selon l'invention est caractérisé en ce que l'on produit simultanément, au sein d'un même milieu, des gènes au moins partiellement composés de polynucléotides synthétiques stochastiques, que l'on introduit les gènes ainsi obtenus dans des cellules-hôtes, que l'on cultive simultanément les souches indépendantes de cellules-hôtes modifiées renfermant ces gênes, de manière à cloner les gènes stochastiques et à provoquer la production des protéines exprimées par chacun de ces gênes stochastiques, que l'on effectue le criblage et/ou la sélection des souches de cellules-hôtes modifiées de façon à identifier les souches produisant des peptides ou polypeptides ayant au moins une propriété donnée, que l'on isole les souches ainsi identifiées et qu'on les cultive de façon à produire au moins un peptide ou polypeptide ayant ladite propriété.

Conformément à un premier mode de mise en oeuvre de ce procédé, on produit les gènes par copolymérisation stochastique des quatre sortes de deoxyphosphonucléotides A, C, G et T à partir des deux extrémités d'un vecteur d'expression préalablement linéarisé, puis formation d'extrémités cohésives de façon à former un premier brin d'ADN stochastique, constitué d'une molécule du vecteur d'expression possédant deux séquences stochastiques dont les extrémités 3' sont complémentaires, suivie de la synthèse du second brin de cet ADN stochastique.

Selon un deuxième mode de mise en oeuvre, on produit les gênes par copolymérisation stochastique d'oligonucléotides sans extrémités cohésives, de façon à former des fragments d'ADN stochastiques, suivie de la ligation de ces fragments à un vecteur d'expression préalablement linéarisé.

Le vecteur d'expression peut être un plasmide, notamment un plasmide bactérien. D'excellents résultats ont été obtenus en utilisant, comme vecteur d'expression, le plasmide pUC8.

Le vecteur d'expression peut également être un ADN viral ou un hybride de plasmide et d'ADN viral.

Les cellules-hôtes peuvent être des cellules procaryotes telles que les cellules HB 101 et C 600, ou des cellules eucaryotes.

Lorsque l'on procède conformément au deuxième mode de mise en oeuvre susmentionné, on peut utiliser des oligonucléotides formant un groupe d'octamères palindromiques.

Des résultats particulièrement bons sont obtenus en utilisant le groupe d'octamères palindromiques suivant :

5' GGAATTCC 3'
5' GGTCGACC 3'
5' CAAGCTTG 3'
5' CCATATGG 3'
5' CATCGATG 3'

On peut également utiliser des oligonucléotides formant un groupe d'heptamères palindromiques.

De très bons résultats sont obtenus en utilisant le groupe d'heptamères palindromiques suivant : 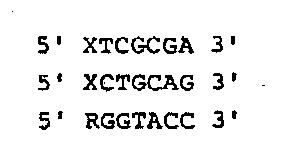 où X = A, G, C ou T et R = A ou T.

Conformément à un mode de mise en oeuvre particulièrement avantageux, l'on isole et purifie l'ADN transformant des plasmides provenant d'une culture de souches indépendantes de cellules-hôtes modifiées obtenues en procédant de la manière spécifiée ci-dessus, puis l'on provoque la coupure de l'ADN purifié au moyen d'au moins un enzyme de restriction correspondant à un site spécifique de coupure enzymatique présent dans ces octamères ou heptamères palindromiques mais absent du vecteur d'expression utilisé, cette coupure étant suivie de l'inactivation de l'enzyme de restriction, et l'on traite ensuite simultanément l'ensemble des fragments d'ADN stochastiques linéarisés, ainsi obtenus, par la T4 DNA ligase, de façon à créer un nouvel ensemble d'ADN contenant des séquences stochastiques nouvelles, ce nouvel ensemble pouvant donc contenir un nombre de gênes stochastiques supérieur au nombre de gènes de l'ensemble initial, et on utilise ce nouvel ensemble d'ADN transformant pour modifier des cellules-hôtes et cloner les gènes, et, finalement, on crible et/ou on sélectionne et on isole les nouvelles souches de cellules-hôtes transformées et finalement, on les cultive de façon à produire au moins un peptide ou polypeptide, par exemple une protéine nouvelle.

La propriété servant de critère de sélection des souches de cellules-hôtes peut être l'aptitude des peptides ou polypeptides, produits par cette souche, à catalyser une réaction chimique donnée.

Par exemple, pour la production d'une pluralité de peptides et/ou polypeptides, ladite propriété peut être l'aptitude à catalyser une séquence de réactions conduisant d'un groupe initial donné de composés chimiques à au moins un composé cible.

En vue de la production d'un ensemble constitué d'une pluralité de peptides et/ou polypeptides réflexivement autocatalytiques, ladite propriété peut être l'aptitude de catalyser la synthèse de cet ensemble lui-même à partir d'acides aminés et/ou d'oligopeptides, dans un milieu approprié.

Ladite propriété peut également être l'aptitude à modifier sélectivement les propriétés chimiques et/ou biologiques d'un composé donné, par exemple l'aptitude à modifier sélectivement l'activité catalytique d'un polypeptide.

Ladite propriété peut aussi être l'aptitude à simuler, inhiber ou modifier au moins une fonction biologique d'au moins un composé biologiquement actif, choisi, par exemple, parmi les hormones, les neurotransmetteurs, les facteurs d'adhésion ou de croissance et les régulateurs spécifiques de la réplication de l'ADN et/ou de la transcription et/ou de la traduction de l'ARN.

Ladite propriété peut être, également, l'aptitude du peptide ou polypeptide à se lier à un ligand donné.

L'invention a également pour objet l'utilisation du peptide ou polypeptide obtenu par le procédé spécifié ci-dessus pour la détection et/ou la titration d'un ligand.

Conformément à un mode de mise en oeuvre particulièrement avantageux, le critère de sélection de la souche de cellules-hôtes modifiée est l'aptitude des peptides ou polypeptides à simuler ou modifier les effets d'une molécule biologiquement active, par exemple une protéine, et l'on effectue le criblage et/ou la sélection de la souche de cellules-hôtes modifiée produisant au moins un peptide ou polypeptide ayant cette propriété en préparant des anticorps contre cette molécule active, et en utilisant ces anticorps, après leur purification, pour identifier les souches contenant ce peptide ou polypeptide, puis en cultivant les souches ainsi identifiées et en séparant et en purifiant le peptide ou polypeptide produit par ces souches, et, finalement, en soumettant ce peptide ou polypeptide à un essai in vitro pour vérifier qu'il présente bien l'aptitude à simuler ou modifier les effets de ladite molécule.

Conformément à un autre mode de mise en oeuvre du procédé selon l'invention, la propriété servant de critère de sélection est d'avoir au moins un épitope semblable à l'un des épitopes d'un antigène donné.

L'invention porte également sur les polypeptides obtenus par le procédé spécifié ci-dessus, et utilisables comme substance active chimiothérapeutique.

En particulier, dans le cas où ledit antigène est l'EGF, l'invention permet l'obtention de polypeptides utilisables pour le traitement chimiothérapeutique des épithéliomes.

Selon une variante du procédé, l'on identifie et isole les souches de cellules-hôtes modifiées produisant les peptides ou polypeptides ayant la propriété désirée par chromatographie d'affinité sur anticorps correspondant à une protéine exprimée par la partie naturelle de l'ADN hybride.

Par exemple, dans le cas où la partie naturelle de l'ADN hybride contient un gène exprimant la β-galactosidase, l'on peut avantageusement identifier et isoler lesdites souches de cellules-hôtes modifiées par chromatographie d'affinité sur anticorps anti- β-galactosidase.

Après expression et purification des peptides ou polypeptides hybrides, on peut séparer et isoler leurs parties nouvelles.

L'invention porte également sur une application du procédé spécifié ci-dessus pour la préparation d'un vaccin, cette application étant caractérisé par le fait que l'on isole des anticorps contre un agent pathogène, par exemple des anticorps formés après injection de cet agent pathogène dans l'organisme d'un animal capable de former des anticorps contre cet agent, et on utilise ces anticorps pour identifier les clones produisant au moins une protéine ayant au moins un épitope semblable à l'un des épitopes de l'agent pathogène, on cultive les souches de cellules-hôtes modifiées correspondant à ces clones, de façon à produire cette protéine, on isole et purifie cette protéine à partir des cultures de ces souches de cellules, et on utilise cette protéine pour la production d'un vaccin contre l'agent pathogène.

Par exemple, pour la préparation d'un vaccin anti-HVB, l'on peut extraire et purifier au moins une protéine de capside de virus HVB, injecter cette protéine dans l'organisme d'un animal capable de former des anticorps contre cette protéine, recueillir et purifier les anticorps ainsi formés, utiliser ces anticorps pour identifier les clones produisant au moins une protéine ayant au moins un épitope semblable à l'un des épitopes du virus HVB, cultiver les souches de cellules-hôtes modifiées correspondant à ces clones, de façon à produire cette protéine, isoler et purifier cette protéine à partir des cultures de ces souches de cellules, et utiliser cette protéine pour la production d'un vaccin anti-HVB.

Avantageusement, conformément à un mode de mise en oeuvre du procédé selon l'invention, les cellules-hôtes consistent en bactéries du genre Escherichia coli dont le génome ne contient ni le gêne naturel exprimant la β-galactosidase, ni le gêne EBG, c'est-à-dire des bactéries E. coli (Z⁻, EBG⁻), on cultive les cellules-hôtes modifiées en présence du milieu X-gal et de l'inducteur IPTG, que l'on détecte, dans le milieu de culture, les clones positifs pour la fonction β-galactosidase, et, finalement, on transplante ensuite cet ADN dans une souche de cellules-hôtes appropriée à la culture en grande quantité en vue de la production industrielle d'au moins un peptide ou polypeptide.

La propriété servant de critère de sélection des souches de cellules-hôtes transformées peut également être l'aptitude, des polypeptides ou protéines, produites par culture de ces souches, à se lier à un composé donné.

Ce composé peut être, notamment, avantageusement choisi parmi les peptides, les polypeptides et les protéines, notamment les protéines régulatrices de l'activité de transcription de l'ADN.

D'autre part, ledit composé peut également être choisi parmi les séquences d'ADN et d'ARN.

L'invention a également pour objet les protéines obtenues dans le cas où la propriété servant de critère de sélection des souches de cellules-hôte transformées consiste précisément dans l'aptitude de ces protéines à se lier à des protéines régulatrices de l'activité de transcription de l'ADN, ou encore à des séquences d'ADN ou d'ARN.

L'invention a, en outre, pour objet l'utilisation d'une protéine obtenue dans le premier cas particulier qui vient d'être mentionné, comme séquence cis-régulatrice de la réplication ou la transcription d'une séquence d'ADN voisine.

D'autre part, l'invention a également pour objet l'utilisation des protéines obtenues dans le second cas particulier susmentionné pour modifier les propriétés de transcription ou de réplication d'une séquence d'ADN, dans une cellule contenant cette séquence d'ADN, et exprimant cette protéine.

L'invention a également pour objet un procédé de production d'ADN, caractérisé par le fait que l'on produit simultanément, au sein d'un même milieu, des gènes au moins partiellement composés de polynucléotides synthétiques stochastiques, que l'on introduit les gènes ainsi obtenus dans des cellules-hôtes, de façon à produire un ensemble de cellules-hôtes modifiées, que l'on effectue un criblage et/ou une sélection de cet ensemble, de façon à identifier les cellules hôtes qui renferment dans leur génome des séquences stochastiques d'ADN présentant au moins une propriété désirée, et l'on isole finalement l'ADN à partir de cultures des cellules-hôtes ainsi identifiées.

L'invention a, en outre, pour objet un procédé de production d'ARN, caractérisé par le fait que l'on produit simultanément, au sein d'un même milieu, des gènes au moins partiellement composés de polynucléotides synthétiques stochastiques, que l'on introduit les gênes ainsi obtenus dans des cellules-hôtes, de façon à produire un ensemble de cellules-hôtes modifiées, que l'on cultive simultanément les souches indépendantes de cellules-hôtes modifiées ainsi produites, que l'on effectue un criblage et/ou une sélection de cet ensemble, de façon à identifier les cellules-hôtes qui renferment des séquences stochastiques d'ABN présentant au moins une propriété désirée, et que l'on isole l'ARN à partir de cultures de cellules-hôtes ainsi identifiées.

Ladite propriété peut être, avantageusement, l'aptitude à se lier à un composé donné, qui peut être, par exemple, un peptide, un polypeptide ou une protéine, ou bien l'aptitude à catalyser une réaction chimique donnée, ou encore la propriété d'être un ARN de transfert.

On va maintenant décrire, plus en détails, le procédé selon l'invention, ainsi que certaines de ses applications, en se référant à des exemples, non limitatifs, de mise en oeuvre.

Tout d'abord, on va décrire des modes opératoires particulièrement avantageux pour effectuer la synthèse de gênes stochastiques et l'introduction de ces gènes dans des bactéries, de manière à produire des souches de bactéries transformées.

### I) Synthèse directe sur un vecteur d'expression

### a) Linéarisation du vecteur

30 µg (c'est-à-dire approximativement 10¹³ molécules) du vecteur d'expression pUC8 sont linéarisés par incubation pendant 2 heures à 37°(avec 100 unités de l'enzyme de restriction PstI dans une volume de 300 µl du tampon standard approprié. Le vecteur linéarisé est traité au phénol-chloroforme puis précipité à l'éthanol, repris dans un volume de 30 µl et chargé sur un gel d'agarose à 0,8 %, en milieu standard TEB. Après migration dans un champ de 3V/cm pendant trois heures, le vecteur linéaire est électro-élué, précipité à l'éthanol et repris dans 30 µl d'eau.

### b) Synthèse stochastique par l'enzyme Terminal-Transférase (TdT)

On fait réagir 30 µg du vecteur linéarisé avec 30 unités de TdT dans 300 µl du tampon approprié, pendant deux heures, à 37°C, en présence de 1mM de dGTP, 1mM de dCTP, 0,3mM de dTTP et 1mM de dATP: On choisit une concentration plus basse de dTTP, dans le but de diminuer la fréquence des codons "stop" dans l'ARN messager correspondant. Un résultat semblable, quoique moins favorable, peut être obtenu en utilisant une concentration plus basse pour le d'ATP que pour les autres deoxynucléotides triphosphates. Le progrès de la réaction de polymérisation sur l'extrémité 3' des sites Pst I est suivi par l'analyse sur gel de parties aliquotes prélevées en cours de réaction.

Lorsque la réaction atteint ou dépasse une valeur moyenne de 300 nucléotides par extrémité 3', elle est interrompue et les nucléotides libres sont séparés du polymère par précipitation différentielle ou par passage sur tamis moléculaire du type Biogel P60. Après concentration par précipitation à l'éthanol, le polymère est soumis séquentiellement à une polymérisation supplémentaire par TdT en présence de dATP d'abord, puis de dTTP. Ces deux dernières réactions sont séparées par une filtration sur gel et conduites pendant des temps courts (30 secondes à 3 minutes), de manière à aboutir à l'addition séquentielle de 10-30A, suivis de 10-30 T à l'extrémité 3' des polymères.

### c) Synthèse du second brin de l'ADN stochastique

Chaque molécule de vecteur possède, à l'issue des opérations précédentes, deux séquences stochastiques dont les extrémités 3' sont complémentaires. Le mélange de polymères est alors incubé dans des conditions favorisant l'hybridation de ces extrémités complémentaires (150 mM Nacl, 10 mM Tris-HCl, pH 7,6, 1 mM EDTA, à 65°C, pendant 10 minutes, puis abaissement de la température jusqu'à 22°C, à raison de 3 à 4°C/heure). Les polymères hybridés sont alors soumis à l'action de 60u du grand fragment de la polymérase I (Klenow) en présence des quatre nucléotides triphosphates (200mM) à 4°C, pendant deux heures. Cette étape réalise la synthèse du second brin à partir de l'extrémité 3' des polymères hybridés. Les molécules résultant de cette synthèse directe à partir d'un vecteur linéarisé sont alors utilisées pour transformer des cellules compétentes.

### d) Transformation des souches compétentes

100 à 200 ml de cellules compétentes HB101 ou C600, à la concentration de 10¹⁰ cellules/ml, sont incubés avec la préparation d'ADN stochastique, en présence de 6mM CaCl₂, 6 mM Tris-Hcl, pH8, 6 mM MgCl₂, pendant 30 minutes, à 0°C. Un choc thermique de 3 minutes à 37°C est imposé au mélange, suivi par l'addition de 400-800 ml de milieu de culture NZY, sans antibiotique. La culture transformée est incubée à 37°C pendant 60 minutes, puis diluée à 10 litres par addition de milieu NZY contenant 40 µg/ml d'ampicilline. Après 3 à 5 heures d'incubation à 37°C, la culture amplifiée est soumise à une centrifugation, et le culot des cellules transformées est lyophilysé et conservé à -70°C. Une telle culture comprend de 3 x 10⁷ à 10⁸ transformants indépendants, contenant chacun un gène stochastique unique inséré dans un vecteur d'expression.

### II) Synthèse de gènes stochastiques à partir d'oligonucléotides sans extrémités cohésives

Ce mode opératoire est fondé sur le fait que la polymérisation d'oligonucléotides palindromiques judicieusement choisis permet d'assembler des gènes stochastiques ne comportant aucun codon "stop" dans chacun des six cadres de lecture possibles, tout en assurant une représentation équilibrée de triplets spécifiant tous les acides aminés. Par ailleurs, et pour éviter la répétition de motifs de séquences dans la protéine résultante, les oligonucléotides penvent comporter un nombre de bases qui n'est pas un multiple de trois. L'exemple suivant décrit l'utilisation d'une combinaison possible d'oligonucléotides qui remplit ces critères :

### a) Choix d'un groupe d'octamères

Le groupe d'oligonucléotides suivant 
5' GGAATTCC 3'
5' GGTCGACC 3'
5' CAAGCTTG 3'
5' CCATATGG 3'
5' CATCGATG 3'
 est composé de 5 palindromes (donc autocomplémentaires) dont il est facile de vérifier que leur polymérisation stochastique n'engendre pas de codon "stop" et spécifie tous les acides aminés.

Bien entendu, on pourrait également utiliser d'autres groupes d'octamères palindromiques n'engendrant pas de codons "stop" et spécifiant tous les acides aminés des polypeptides. On comprendra également que l'on pourrait utiliser des groupes d'octamères non palindromiques à condition d'employer aussi leurs compléments par la formation de segments bicaténaires.

### b) Assemblage d'un gène stochastique à partir d'un groupe d'octamères

On fait réagir un mélange comportant 5 µg de chacun des oligonucléotides indiqués ci-dessus (préalablement phosphorylés en 5' par un procédé connu en soi) dans un volume de 100 µl contenant 1 mM ATP, 10 % de polyéthylèneglycol et 100 u de T₄ DNA ligase, dans le tampon approprié, à 13°C, pendant six heures. Cette étape effectue la polymérisation stochastique des oligomères sous forme bi-caténaire, sans extrémités cohésives. On isole alors par passe sur tamis moléculaire (Biogel P60) les polymères résultant de l'assemblage de 20 à 100 oligomères. Après concentration, cette fraction est à nouveau soumise à la polymérisation catalysée par la T4 DNA ligase dans les conditions décrites ci-dessus. On isole alors, comme décrit plus haut, les polymères résultant de l'assemblage d'au moins 100 oligomères.

### c) Préparation du plasmide-hôte

Le vecteur d'expression pUC8 est linéarisé par l'enzyme Sma I dans le tampon appraprié, comme décrit ci-dessus. Le vecteur linéarisé par l'enzyme Sma I ne comporte pas d'extrémités cohésives. Le vecteur linéarisé est alors traité par la phosphatase alcaline d'intestin de veau (CIP) à raison d'une unité par µg de vecteur dans le tampon approprié, à 37°C, pendant 30 minutes. L'enzyme CIP est alors inactivée par deux extractions successives au phénolchloroforme. Le vecteur linéarisé et déphosphorylé est précipité à l'éthanol puis repris dans l'eau à 1 mg/ml.

### d) Ligation des gênes stochastiques au vecteur

Des quantités équimolaires de vecteur et de polymère sont mélangées et incubées en présence de 1000 u de T4 DNA ligase, 1 mM ATP, 10 % de polyéthylène glycol dans le tampon approprié, pendant 12 heures, à 13°C. Cette étape réalise la ligation des polymères dans le vecteur d'expression et engendre des molécules bicaténaires, circulaires et, par conséquent, transformantes.

### Transformation des souches compétentes

On procède à la transformation des souches compétentes de la manière précédemment décrite.

### III) Assemblage d'un gène stochastique à partir d'un groupe d'heptamères

Ce mode opératoire se distingue de celui qui vient d'être décrit par le fait qu'il utilise des heptamères palindromiques comportant une extrémité cohésive variable, au lieu d'octamères. Il présente l'avantage de permettre l'assemblage de séquences stochastiques comportant une plus faible proportion de motifs identiques.

### a) Choix d'un groupe d'heptamères

On peut utiliser, par exemple, les 3 heptamères palindromiques suivants : 
5' XTCGCGA 3'
5' XCTGCAG 3'
5' RGGTACC 3'
 pour lesquels X = A, G, C ou T et R = A ou T et dont la polymérisation ne peut engendrer de codons "stop" et comporte des triplets spécifiant tous les acides aminés.

Bien entendu, on pourrait également utiliser tout autre groupe d'heptamères remplissant ces mêmes conditions.

### b) Polymérisation d'un groupe d'heptamères

Cette polymérisation s'effectue exactement de la manière précédemment décrite dans le cas des octamères.

### c) Elimination des extrémités cohésives

Les polymères ainsi obtenus comportent une base non appariée à leur deux extrémités 5'. Il est donc nécessaire d'additionner la base complémentaire aux extrémités 3' correspondantes. Ceci s'effectue de la manière suivante : on fait réagir 10 µg de polymères double brin avec 10 u d'enzymes de Klenow, en présence des quatre déoxynucléotidephosphates (200 mM) dans un volume de 100 µl, à 4°C, pendant 60 minutes. L'enzyme est inactivée par extraction au phénol-chloroforme et les polymères sont débarrassés des nucléotides libres résiduels par précipitation différentielle. Les polymères sont alors ligasés au plasmide-hôte (préalablement linéarisé et déphosphorylé) en procédant de la manière décrite ci-dessus.

Il est à remarquer que les deux derniers modes opératoires qui viennent d'être décrits utilisent des octamères ou heptamères palindromiques qui constituent des sites spécifiques d'enzymes de restriction. Ces sites sont absents pour la plupart du vecteur d'expression pUC8. Il est donc possible d'augmenter considérablement la complexité d'une préparation initiale de gènes stochastiques en procédant de la manière suivante ; on prépare l'ADN des plasmides provenant d'une culture de 10⁷ transformants indépendants obtenus par l'un des deux derniers modes opératoires décrits ci-dessus. A cet ADN purifié, on fait alors subir une digestion partielle par l'enzyme de restriction Cla I (mode opératoire II) ou par l'enzyme de restriction Pst I (mode opératoire III). Après inactivation de l'enzyme, l'ADN partiellement digéré est traité à la T4 DNA ligase, ce qui a pour effet de créer un nombre extrêmement grand de séquences nouvelles qui conservent les propriétés fondamentales des séquences initiales. Ce nouvel ensemble de séquences stochastiques est alors utilisé pour transformer des cellules compétentes.

Par ailleurs, les gênes stochastiques clonés en procédant conformément aux modes opératoires II et III peuvent être excisés intacts du vecteur d'expression pUC8 en utilisant les sites de restriction propres au vecteur de clonage et non représenté dans l'ADN stochastique.

La recombinaison au sein des gênes stochastiques engendrés par les deux derniers modes opératoires qui viennent d'être décrits, recombinaison qui résulte de l'homologie interne et des motifs de récurrence, fournit une importante méthode additionnelle de mutagenèse in vivo des séquences codantes. Il en résulte une augmentation du nombre des gênes nouveaux qui peuvent être examinés.

Finalement, pour tout processus de génération de gènes synthétiques nouveaux, on peut utiliser de nombreuses techniques usuelles de modification des gênes in vivo ou in vitro, telles que changement du cadre de lecture, inversion des séquences par rapport à leur promoteur, ou utilisation de souches-hôtes exprimant un ou plusieurs tRNA suppresseurs.

En se référant à la partie de la description qui précède, on comprendra qu'il est possible de construire in vitro un nombre extrêmement grand (par exemple, supérieur à un milliard) de gênes différents, par polymérisation enzymatique de nucléotides et d'oligonucléotides. Cette polymérisation s'effectue selon un processus stochastique déterminé par les concentrations respectives des nucléotides ou oligonucléotides présents dans le milieu de réaction.

Comme indiqué ci-dessus, deux méthodes peuvent être utilisées pour cloner de tels gènes (ou séquences codantes) : la polymérisation peut s'effectuer directement sur un vecteur de clonage d'expression, préalablement linéarisé, ou bien on peut choisir de procéder séquentiellement à la polymérisation puis à la ligation des polymères au vecteur de clonage et d'expression.

Dans les deux cas, on procède ensuite à la transformation ou à la transfection de cellules bactériennes compétentes (ou de cellules en culture). Cette étape réalise le clonage des gênes stochastiques dans des cellules vivantes où ils sont indéfiniment propagés et exprimés.

Bien entendu, outre les modes opératoires qui viennent d'être décrits, on pourrait également employer toute autre méthode appropriée pour la synthèse de séquences stochastiques. En particulier, on pourrait procéder à la polymérisation, par voie biochimique, d'oligomères monocaténaires d'ADN ou d'ARN obtenus par synthèse chimique, puis traiter ces segments d'ADN ou d'ARN par des procédés connus en soi de préparation de copies d'ADN (c ADN) bicaténaire en vue du clonage des gènes.

### Criblage ou sélection des souches de cellules-hôtes modifiées

L'étape ultérieure du procédé selon l'invention consiste à examiner les cellules transformées ou transfectées, par sélection ou criblage, en vue d'isoler une ou plusieurs cellules dont l'ADN transformant ou transfectant conduise la synthèse d'un produit de transcription (ARN) ou de traduction (protéine) possédant une propriété souhaitée. Ces propriétés peuvent être, par exemple, enzymatiques, fonctionnelles ou structurelles.

Une des particularités les plus remarquables du procédé selon l'invention est de permettre le criblage ou la sélection simultanée d'un produit exploitable (ARN ou protéine) et de son gène producteur. De surcroît, l'ADN synthétisé et cloné comme décrit peut être sélectionné ou criblé en vue d'isoler des séquences d'ADN constituant un produit en soi, doté de propriétés biochimiques exploitables.

On va maintenant décrire, à titre d'exemples non limitatifs, des modes opératoires préférentiels pour le criblage ou la sélection de souches de cellules transformées ainsi que des protéines nouvelles présentant un intérêt en vue d'applications industrielles ou médicales.

L'un de ces modes opératoires résulte de l'idée de produire une série d'anticorps polyclonaux ou monoclonaux, obtenus de manière connue en soi, dirigée contre une protéine ou un autre type de molécule d'intérêt biochimique ou médical, cette molécule étant, ou pouvant être rendue, immunogénique, et d'utiliser ces anticorps comme sonde pour identifier parmi de très nombreux clones transformés par des gènes stochastiques ceux qui réagissent avec ces anticorps. Cette réaction résulte de l'homologie de structure existant entre le polypeptide synthétisé par le gène stochastique et la molécule initiale. On peut ainsi isoler de nombreuses protéines nouvelles se comportant comme des épitopes ou déterminants antigéniques de la molécule initiale. De telles protéines nouvelles sont susceptibles de simuler, stimuler, moduler ou bloquer l'effet de la molécule initiale. On comprendra que ce mode de sélection ou de criblage est, en lui-même, susceptible d'avoir de très nombreuses applications pharmacologiques et bio-médicales. On va maintenant décrire, à titre d'exemple non limitatif, ce premier mode opératoire en relation avec un cas particulier:

L'EGF (epidermal growth factor) est une petite protéine présente dans le sang et dont le rôle est de stimuler la croissance des cellules épithéliales. Cet effet est obtenu par l'interaction de l'EGF avec un récepteur spécifique situé dans la membrane des cellules épithéliales.

On prépare des anticorps dirigés contre l'EGF par injection chez l'animal d'EGF couplé à KLH (keyhole limpet hemocyanin) pour augmenter l'immunogénicité de l'EGF. Les anticorps anti-EGF des animaux immunisés sont purifiés, par exemple par passage sur une colonne d'affinité, dont le ligand est l'EGF ou un peptide de synthèse correspondant à un fragment d'EGF. Les anticorps anti-EGF purifiés sont utilisés comme sonde pour le criblage d'un grand nombre de clones bactériens lysés au chloroforme sur support solide. Les anticorps anti-EGF se combinent aux peptides ou protéines stochastiques dont les épitopes ressemblent à ceux de l'antigène initial. Les clones contenant ces peptides ou protéines sont mis en évidence par autoradiographie après incubation des supports solides avec de la protéine A radioactive ou après incubation avec un anticorps antianticorps radioactif.

Ces étapes identifient les clones contenant chacun une protéine (et son gêne) réagissant avec l'anticorps de criblage. On peut ainsi faire des criblages parmi un très grand nombre de souches cellulaires bactériennes ou de plaques virales (par exemple de l'ordre de un million) et il est possible de détecter des quantités extrêmement faibles, par exemple de l'ordre de 1 ng, de protéine produite. On procède alors à la culture des clones identifiés puis à la purification des protéines détectées par les moyens conventionnels. Les protéines purifiées sont testées in vitro dans des cultures de cellules épithéliales pour déterminer si elles inhibent, simulent ou modulent l'effet sur ces cultures de l'EGF. Certaines des protéines obtenues par ce moyen sont susceptibles d'être utilisées pour le traitement chimiothérapique des épithéliomes. Les activités des protéines ainsi obtenues peuvent être améliorées par mutation de l'ADN codant pour les protéines, de manière analogue à celle décrite ci-dessus. Une variante de ce mode opératoire consiste à purifier des peptides, polypeptides ou protéines stochastiques, pouvant être utilisées comme vaccins ou plus généralement pouvant être utilisées pour conférer une immunité contre un agent pathogène ou pour exercer d'autres effets sur le système immunologique, par exemple, créer une tolérance ou diminuer l'hypersensibilité à l'égard d'un antigène donné, notamment par suite de la combinaison de ces peptides, polypeptides ou protéines avec les anticorps dirigés contre cet antigène. On comprendra que l'on peut utiliser ainsi ces peptides, polypeptides ou protéines aussi bien in vitro qu'in vivo.

Plus précisément, par exemple, dans l'ensemble des protéines nouvelles qui réagissent avec l'anticorps contre un antigène donné X, chacune a au moins un épitope en commun avec X, donc l'ensemble a un ensemble d'épitopes commun avec X. Ceci permet d'utiliser l'ensemble ou un sous-ensemble comme vaccin pour conférer l'immunité contre X. Il est, par exemple, aisé de purifier une ou plusieurs des protéines de capside du virus de l'hépatite B. Ces protéines sont injectées chez l'animal, par exemple le lapin, et on recueille les anticorps correspondant à l'antigène de départ par purification sur colonne d'affinité. On utilise ces anticorps, de la manière décrite ci-dessus, pour identifier les clones produisant une protéine ayant un épitope semblable à l'un au moins des épitopes de l'antigène initial. Après purification, on utilise ces protéines comme antigène (soit isolément, soit en combinaison) dans le but de conférer une protection contre l'hépatite B. La production ultérieure du vaccin ne nécessite plus le recours à l'agent pathogène initial.

Il est à remarquer que, lors de la description des modes opératoires qui précèdent, un certain nombres de manières de procéder à la sélection et au criblage ont été décrits. Tous ces modes opératoires impliquent la purification d'une protéine particulière à partir d'une souche transformée. Ces purifications de protéines peuvent être effectuées conformément aux méthodes usuelles et font appel, en particulier, aux techniques de chromatographie sur gel, sur échangeur d'ion, et à la chromatographie d'affinité. Par ailleurs, les protéines issues de gènes stochastiques peuvent avoir été clonées sous forme de protéines hybrides, comportant, par exemple, une séquence de l'enzyme β-galactosidase permettant la chromatographie d'affinités sur anticorps anti-β-galactosidase et permettant le clivage subséquent de la partie hybride (c'est-à-dire permettant de séparer la partie nouvelle de la partie bactérienne).On va maintenant décrire le principe et la mise en oeuvre de la sélection des peptides ou polypeptides et des gènes correspondants, exprimant ces peptides ou polypeptides, conformément à une deuxième méthode de criblage ou sélection fondée sur la détection de l'aptitude de ces peptides ou polypeptides à catalyser une réaction spécifique.

A titre d'exemple concret non limitatif, on va décrire la mise en oeuvre du criblage ou de la sélection dans le cas particulier de protéines capables de catalyser le clivage du lactose, ce qui est normalement une fonction remplie par l'enzyme β-galactosidase (β-gal).

Comme décrit ci-dessus, la première étape du procédé consiste à engendrer un très grand ensemble de vecteurs d'expression exprimant chacun une protéine nouvelle distincte. De manière concrète, on peut, par exemple, choisir le vecteur d'expression pUC8 avec clonage de séquences stochastiques d'ADN au site de restriction Pst I.
Les plasmides ainsi obtenus sont ensuite introduits dans une souche de E. coli dans le génome de laquelle on a éliminé par les méthodes génétiques conventionnelles le gène naturel pour la β-galactosidase, Z, et un deuxième gène EBG, sans rapport avec le le premier, mais capable de muter vers la fonction β-gal. De telles cellules-hôtes (Z⁻, EBG⁻) ne sont pas capables par elles-mêmes de catalyser l'hydrolyse du lactose et, par conséquent, d'utiliser le lactose comme source de carbone pour la croissance. Ceci permet d'utiliser cette souche hôte pour le criblage ou la sélection de la fonction β-gal.

Une méthode d'essai biologique qui convient pour l'étude des souches transformées de E. coli présentant des gènes nouveaux exprimant la fonction β-gal consiste dans la culture des bactéries ainsi transformées dans des boîtes de Pétri renfermant le milieu X-gal. Dans ce cas, toute colonie bactérienne exprimant une fonction β -gal est visualisée sous forme d'une colonie bleue. En utilisant un tel essai biologique, on peut détecter même une activité catalytique faible. L'activité spécifique des enzymes les plus caractéristiques s'établit entre 10 et 10 000 molécules de produit par seconde.

En supposant qu'une protéine synthétisée par un gène stochastique présente une activité spécifique faible, de l'ordre de une molécule par 100 secondes, il serait toujours possible de détecter une telle activité catalytique. Dans une boîte de Pétri renfermant le milieu X-gal, en présence de l'inducteur non métabolisable IPTG (isopropyl--D-thiogalactoside) la visualisation d'une région bleue requiert le clivage d'environ 10¹⁰ à 10¹¹ molécules de X-gal par millimètre carré. Une colonie bactérienne exprimant un enzyme faible et occupant une superficie de 1mm² contient environ 10⁷ à 10⁸ cellules. Si chaque cellule présente une seule copie de l'enzyme faible, chaque cellule doit catalyser entre 10 000 et 100 clivages de X-gal afin de pouvoir être détectée, ce qui prend environ 2,7 à 270 heures. Etant donné que, dans des conditions sélectives, on peut s'attendre à une amplification du nombre des copies de plasmides par cellules, par exemple de 5 à 20 copies par cellule et même de 100 à 1000, et compte tenu du fait que jusqu'à 10% des protéines de la cellule peuvent être spécifiées par le nouveau gène, la durée nécessaire à la détection d'une colonie bleue dans le cas de 100 molécules d'enzymes de faible activité spécifique par cellule serait de l'ordre de 0,27 heure à 2,7 heures.

Par conséquent, le criblage d'un grand nombre de colonies bactériennes indépendantes, exprimant chacun un gène nouveau différent, en prenant comme critère de sélection la faculté d'exprimer la fonction β-gal est parfaitement réalisable. On peut ainsi réaliser le criblage d'environ 2000 colonies dans une boîte de Pétri ordinaire ayant 10 cm de diamètre. Par conséquent, on peut cribler environ 20 millions de colonies sur une feuille d'agar X-gal de 1 m².

Il est à remarquer que les colonies bactériennes apparaissant en bleu sur les boîtes de Pétri X-gal pourraient être faussement positives en raison d'une mutation dans le gènome bactérien lui conférant la capacité de métaboliser le lactose, ou pour d'autre raisons que celles qui résultent de l'activité catalytique de la protéine nouvelle exprimée par les cellules de la colonie. De tels faux positifs peuvent être directement éliminés en purifiant l'ADN des vecteurs d'expression provenant des colonies positives et en retransformant les cellules-hôtes E. Coli Z⁻,EBG⁻. Si l'activité β-gal résulte de la nouvelle protéine codée par le nouveau gène dans le vecteur d'expression, toutes les cellules transformées par ce vecteur devraient présenter la fonction β-gal. Au contraire, si la colonie bleue initiale résulte d'une mutation dans le génome de la cellule hôte, il s'agit d'un événement exceptionnel indépendant de la transformation et le nombre des cellules de la nouvelle souche transformée E. Coli servant d'hôte susceptible d'exprimer la fonction β-gal devrait être petit ou nul.

On insistera particulièrement sur la puissance de la purification en masse simultanée des vecteurs d'expression pour tous les clones positifs (bleus) suivie par la retransformation des bactéries naïves. Supposons que l'on désire effectuer un criblage en vue de sélectionner des protéines ayant une fonction catalytique et que la probabilité qu'un nouveau peptide ou polypeptide remplisse cette fonction, au moins faiblement, soit de 10⁻⁶, alors que la probabilité que la souche microbienne hôte E. Coli est sujette à une mutation ayant pour effet qu'elle devienne capable de remplir cette fonction, soit de 10⁻⁵, on peut calculer que sur 20 millions de bactéries transformées soumises au criblage, 20 clones positifs seront, en moyenne, attribuables aux gênes nouveaux sur les vecteurs d'expression que chacune comporte, alors que 200 clones positifs résulteront de mutations d'arrière-plan. La purification en masse des vecteurs d'expression de l'intégralité des 220 clones bactériens positifs et la retransformation des bactéries naïves avec les vecteurs d'expression mis en commun produira un grand nombre de clones positifs constitués de toutes les bactéries transformées avec les 20 vecteurs d'expression qui codent les nouvelles protéines ayant la fonction désirée, et un très petit nombre de clones bactériens résultant de mutations d'arrière-plan contenant les 200 vecteurs d'expression restant sans intérêt. Un petit nombre de cycles de purification de vecteurs d'expression dans les colonies bactériennes positives, ainsi que de retransformation, permet la détection des très rares vecteurs d'expression véritablement positifs par rapport à une activité catalytique désirée, malgré un bruit de fond très élevé de mutations des cellules-hôtes pour cette fonction.

A la suite d'opérations de criblage de ce genre, on peut purifier la nouvelle protéine grâce à la mise en oeuvre de techniques usuelles. La production de cette protéine en grande quantité est rendue possible grâce au fait que l'identification de la protéine utile s'accompagne de l'identification simultanée du gêne codant cette protéine. Par conséquent, on peut utiliser le vecteur d'expression lui-même ou bien on peut transplanter le gène nouveau dans un vecteur d'expression plus approprié à la synthèse et à l'isolation en grande quantité.
On peut appliquer des modes de criblage de ce genre à toute fonction enzymatique pour laquelle il existe un essai biologique approprié. De tels criblages ne nécessitent pas que la fonction enzymatique que l'on recherche soit profitable à la cellule hôte. On peut effectuer un criblage non seulement par rapport à une fonction enzymatique mais également pour toute autre propriété désirée pour laquelle on peut établir un essai biologique approprié. On peut ainsi effectuer même dans le cas simple de la fonction β-gal visualisée sur boîte de Pétri à milieu X-gal, le criblage d'un nombre de gènes nouveaux de l'ordre de 100 millions ou même d'un milliard pour une activité catalytique ou une autre propriété désirée.

### Sélection des cellules-hôtes modifiées

D'autre part, on peut utiliser des techniques de sélection pour toute propriété, catalytique ou autre, dont la présence ou l'absence peut être rendue essentielle à la survie des cellules-hôtes contenant les vecteurs d'expression codant les gènes nouveaux ou pouvant être utiles pour la sélection de virus codant et exprimant le gène nouveau. A titre d'exemple concret, non limitatif, on va maintenant revenir sur la description du mode de sélection en relation avec la fonction β-galactosidase. Une souche appropriée Z⁻EBG⁻ d'E. Coli ne peut pas croître en utilisant le lactose comme seule source de carbone. Ainsi, après la mise en oeuvre de la première étape décrite ci-dessus, on peut cultiver un très grand nombre d'hôtes transformés par des vecteurs d'expression codant les gènes nouveaux, la culture étant effectuée dans des conditions sélectives, soit en diminuant progressivement la concentration des autres sources de carbone, soit en utilisant seulement le lactose dès le départ. Au cours d'une telle sélection. la mutagénèse in vivo par voie de recombinaison ou par récupération explicite de vecteurs d'expression et mutagénèse in vitro de leurs gènes nouveaux par des mutagènes variés, ou par tout autre technique usuelle, permet des améliorations adaptatives de l'aptitude à remplir la fonction catalytique désirée. Lorsqu'il existe à la foi des techniques de sélection et des techniques commodes d'essai biologique, comme dans le cas du présent exemple, on peut utiliser initialement des techniques de sélection pour enrichir la représentation en bactéries hôte exprimant la fonction β-gal, puis effectuer un criblage sur boîte de Pétri à milieu X-gal afin d'identifier efficacement des cellules positives. En l'absence de technique d'essai biologique convenable, l'application de conditions de sélection de plus en plus rigoureuses constitue la voie la plus facile pour purifier un type ou un petit nombre de types de cellules-hôtes distinctes dont les vecteurs d'expression codent les protéines catalysant la réaction choisie.

On peut utiliser ces techniques pour trouver de nouvelles protéines ayant une grande variété de caractéristiques structurelles ou fonctionnelles en plus de l'aptitude à catalyser des réactions spécifiques. Par exemple, on peut effectuer un criblage ou une sélection pour trouver de nouvelles protéines se fixant sur des sites cis-régulateurs de l'ADN et bloquant de ce fait l'expression d'une fonction des cellules hôtes, ou encore bloquant la transcription de l'ADN, stimulant la transcription, etc.

Par exemple, dans le cas de E. Coli une souche mutante du répresseur de l'opéron lactose (i-) exprime de manière constitutive la fonction β-gal en raison du fait que l'opérateur lactose n'est pas réprimé. Toutes les cellules de ce genre produisent des clones bleus sur les boîtes de Pétri contenant le milieu X-gal. Il est possible de transformer de telles souches hôtes avec des vecteurs d'expression synthétisant des protéines nouvelles et effectuer un criblage sur boîte de Pétri à milieu X-gal en vue de détecter les clones qui ne sont pas bleus. Parmi ceux-ci, certains représentent des cas où la nouvelle protéine se fixe sur l'opérateur lactose et réprime la synthèse de β-gal. On peut purifier en masse de tels plasmides, les retransformer , isoler les clones qui ne produisent pas la β-gal et effectuer ensuite une vérification détaillée.

Comme il est mentionné plus haut, le procédé peut être utilisé aux fins de créer puis d'isoler non seulement des protéines exploitables mais aussi des ARN et des ADN constituant des produits en soi, dotés de propriétés exploitables. Ceci résulte évidemment du fait que d'une part, le procédé consiste à créer des séquences stochastiques d'ADN, susceptibles d'interagir directement avec d'autres constituants cellulaires ou biochimiques, et que d'autre part ces séquences clonées dans un vecteur d'expression sont transcrites en ARN eux aussi capables de multiples interactions biochimiques.

### Exemple de mise en oeuvre du procédé pour la création et la sélection d'un ADN exploitable en soi :

Cet exemple illustre la sélection d'un ADN exploitable et le purification et l'étude du mécanisme d'action des protéines régulatrices se liant à l'ADN.
Soit une préparation du récepteur de l'oestradiol, une protéine obtenue par une méthode connue en soi. En présence d'oestradiol, une hormone stéroïde sexuelle, ce récepteur change de conformation et se lie fortement à certaines séquences spécifiques de l'ADN génomique, affectant ainsi la transcription de gênes impliqués dans la différenciation sexuelle et le contrôle de la fertilité.
En incubant une mélange constitué d'oestradiol, de son récepteur et d'un grand nombre de séquences stochastiques différentes insérées dans leur vecteur puis en filtrant le mélange à travers une membrane de nitro cellulose on obtient une sélection directe des séquences stochastiques se liant au complexe oestrogène - récepteur puisque seuls les ADN liés à une protéine sont retenus par la membrane. Après lavage et élution, l'ADN libéré de la membrane est utilisé tel quel pour transformer des bactéries. Après culture des bactéries transformées, on purifie à nouveau les vecteurs qu'elles contiennent et on procède à un ou plusieurs cycles d'incubation, filtration et transformation comme décrit ci-dessus. Ces opérations permettent d'isoler des séquences stochastiques d'ADN dotées d'une affinité élevée pour le complexe oestradiol-récepteur. De telles séquences sont susceptibles de nombreuses applications diagnostiques et pharmacologiques, en particulier pour la mise au point d'oestrogènes de synthèse pour le contrôle de la fertilité et le traitement de la stérilité.

### Création et sélection d'un ARN exploitable en soi

Soit un grand nombre de séquences d'ADN stochastique produites comme il a été décrit et clonées dans un vecteur d'expression. Il va de soi que l'ARN transcrit à partir de ces séquences dans des cellules-hôtes transformées peut être un produit exploitable en soi.
A titre d'exemple non limitatif, on peut sélectionner un gène stochastique codant pour un ARN de transfert (t-ARN) suppresseur par la procédure suivante :

On transforme par un grand nombre (≥ 10⁸) de séquences stochastiques, une souche bactérienne compétente comportant une mutation "nonsense" dans le gène arg E. On étale les bactéries transformées sur milieu minimal sans arginine et contenant l'antibiotique de sélection du plasmide (ampicilline s'il s'agit du vecteur pUC8).Seules les bactéries transformées qui sont devenues capables de synthétiser l'arginine pourront croître. Ce phénotype peut résulter soit d'une mutation en retour soit de l'introduction dans la cellule d'un suppresseur. Il est facile de tester chaque colonie transformée pour déterminer si le phénotype arg⁺ résulte ou non de la présence du gène stochastique dans son vecteur : on se contente de préparer le plasmide de cette colonie et de vérifier qu'il confère le phénotype Arg⁺ à toute cellule arg E qu'il transforme.

### Sélection de protéines capables de catalyser une séquence de réaction

On va maintenant décrire un autre mode de sélection, qui est susceptible d'applications indépendantes, fondé sur le principe de la sélection simultanée, en parallèle, d'un certain nombre de nouvelles protéines capables de catalyser une séquence de réactions connectées entre elles.

L'idée mère de cette méthode est la suivante : étant donné un ensemble de composés chimiques de départ considérés comme des "briques" ou des éléments de construction à partir desquels on désire effectuer la synthèse d'un ou de plusieurs composés chimiques désirés par l'intermédiaire d'une séquence de réactions chimiques catalysées, il existe un très grand nombre de voies de réaction pouvant se substituer les unes aux autres complètement ou en partie, qui sont toutes possibles du point de vue thermodynamique et qui conduisent de l'ensemble des "briques" ou blocs de construction au composé chimique cible désiré. Une synthèse efficace d'un composé cible est favorisée si chaque étape d'au moins l'un des trajets réactionnels menant de l'ensemble des "blocs de construction" au composé cible est constitué de réactions qui sont chacune catalysées. Toutefois, il est relativement moins important de déterminer parmi les nombreux chemins réactionnels complètement ou partiellement indépendants ceux qui bénéficient de la catalyse, Dans la description qui précède, on a montré comment il était possible d'obtenir un très grand nombre de cellules hôtes qui expriment chacune une protéine nouvelle distincte.
Chacune de ces protéines nouvelles est susceptible de catalyser l'une quelconque des réactions possibles dans l'ensemble de toutes les réactions possibles conduisant de l'ensemble des blocs de construction au composé cible. Si un nombre de protéines stochastiques suffisamment grand est présent dans un mélange réactionnel contenant les composés constituant les blocs de construction, de sorte qu'un nombre suffisamment élevé des réactions possibles se trouve catalysé, il y a une forte probabilité qu'une séquence de réactions connectées entre elles pour conduire de l'ensemble de blocs de construction au composé cible sera catalysée par un sous-ensemble des nouvelles protéines. Il est évident que le procédé peut s'étendre à la catalyse non seulement d'un, mais de plusieurs composés cibles, simultanément.

En se fondant sur le principe qui vient d'être énoncé, on peut procéder de la manière suivante pour sélectionner en parallèle un ensemble de nouvelles protéines catalysant une séquence désirée de réactions chimiques :
1. Spécifier l'ensemble désiré de composés constituant les "blocs de construction" en utilisant de préférence un nombre raisonnablement élevé d'espèces chimiques distinctes afin d'augmenter le nombre de voies potentielles concurrentes menant au composé chimique cible désiré.
2. Dans un volume approprié d'une solution de milieu réactionnel, ajouter un nombre très grand de nouvelles protéines stochastiques isolées à partir des cellules transformées ou transfectées synthétisant ces protéines. Effectuer un essai pour déterminer si le composé cible est formé. Si c'est le cas, confirmer que cette formation nécessite la présence du mélange des protéines nouvelles, Si c'est le cas, ce mélange doit contenir un sous-ensemble de protéines catalysant une ou plusieurs séquences de réaction menant de l'ensemble de "blocs de construction" au composé cible. Purifier en divisant la réserve initiale de clones qui synthétisent l'ensemble de nouvelles protéines. stochastiques, le sous-ensemble nécessaire pour catalyser la séquence de réactions conduisant au produit cible.

Plus précisément, à titre d'exemple non limitatif, on va décrire la sélection d'un ensemble de nouvelles protéines aptes à catalyser la synthèse d'un petit peptide spécifié, à savoir un pentapeptide, à partir d'un ensemble de blocs de construction constitué de peptides plus petits et d'acides aminés. Tout peptide est constitué par une séquence linéaire de 20 types différents d'acides aminés, orientée de son extrémité amino à son extrémité carboxe. Tout peptide peut être formé en une étape par condensation terminale de deux peptides plus petits (ou de deux acides aminés), ou par hydrolyse d'un peptide plus grand. Un peptide comprenant M résidus pourra donc être formé d'un nombre égal à M -1 réactions de condensation. Le nombre de réactions, R, par lequel un ensemble de peptides ayant une longueur de 1, 2, 3... M résidus, peut être interconverti est plus grand que le nombre d'espèces moléculaires T. Ceci s'exprime R/_{T} ≃ M-2. Ainsi, en partant d'un ensemble donné de peptides, un grand nombre de chemins réactionnels indépendants ou partiellement indépendants mène à la synthèse d'un pentapeptide cible spécifique. On peut choisir un pentapeptide dont la présence peut être commodément décelée grâce à un essai effectué par des techniques usuelles, par exemple par analyse HPLC (chromatographie en phase liquide sous haute pression), chromatographie sur papier, etc. La formation de la liaison peptidique requiert de l'énergie en milieu aqueux dilué mais, si les peptides participant aux réactions de condensation sont suffisamment concentrés, c'est la formation de la liaison peptidique plutôt que l'hydrolyse qui se trouve thermodynamiquement favorisée et qui se produit avec un rendement élevé en présence d'un catalyseur enzymatique approprié, par exemple la pepsine ou la trypsine, sans nécessiter la présence d'ATP ou d'autres composés à haute énergie. On peut utiliser un tel mélange réactionnel de peptides de petite taille et dont les acides aminés sont marqués radioactivement, au moyen de traceurs radioactifs du type ³H, ¹⁴C, ³⁵S, pour constituer l'ensemble de blocs de construction à concentration suffisamment élevée pour conduire aux réactions de condensation.

On peut, par exemple, procéder de la manière suivante :
on dissout environ 15mg de chaque acide aminé et petit peptide ayant 2 à 4 acides aminés, choisis pour constituer l'ensemble de blocs de construction, dans un volume de 0,25 ml à 1,0 ml de tampon phosphate à 0,1M, pH 7,6. On purifie un grand nombre de protéines nouvelles engendrées et isolées comme décrit ci-dessus à partir de leur hôte bactérien ou autre. On dissout le mélange de ces nouvelles protéines jusqu'à une concentration totale finale de l'ordre de 0,8 à 1,0 mg/ml, dans le même tampon. On ajoute 0,25 ml à 0,5 ml du mélange de protéines au mélange de blocs de construction. On incube à une température de 25°C à 40°C pendant 1 à 40 heures. On enlève des parties aliquotes de 8 ul à intervalles réguliers, dont le premier compte comme "témoin à blanc", avant l'adjonction du mélange de nouvelles protéines. On soumet les échantillons à une analyse par chromatographie en utilisant comme solvant un mélange de n-butanol-acide acétique-pyridine-eau (30:6:20:24 en volume). On sèche le chromatogramme et on le révèle à la ninhydrine ou on l'autoradiographie (avec ou sans écran d'intensification). Du fait que les composés constituant les éléments de construction sont marqués radioactivement, le composé cible sera radioactif et il aura une activité spécifique élevée permettant sa détection au niveau de 1 à 10 ng. Au lieu de l'analyse par chromatographie habituelle, on peut effectuer une analyse par EPLC (high pressure liquid chromatography) qui est plus rapide et plus simple à effectuer. De manière générale, on peut utiliser les procédés d'analyses usuels. De la sorte, on peut détecter un rendement en composés cibles inférieur à une partie par million en poids par rapport aux composés ou "matériaux de construction" initiaux.

Dans le cas où le pentapeptide est formé dans les conditions décrites ci-dessus mais où sa formation n'a pas lieu lorsqu'on utilise un extrait purifié comme ci dessus mais obtenu à partir de cellules transformées par le vecteur d'expression dépourvu d'insertion stochastique, la formation du pentapeptide ne résulte pas de la présence des contaminants bactériens et requiert donc la présence d'un sous-ensemble de nouvelles protéines dans le mélange réactionnel.

L'étape suivante consiste dans la séparation du sous-ensemble particulier de cellules qui contiennent les vecteurs d'expression des nouvelles protéines catalysant la séquence de réactions conduisant au pentapeptide cible. Par exemple si le nombre de réactions formant cette séquences est égal à 5, il y a environ 5 protéines nouvelles qui catalysent les réactions nécessaire. Dans le cas où la "banque de clones" de bactéries contenant les vecteurs d'expression codant les gènes nouveaux contient un nombre de gênes nouveaux distincts de l'ordre de 1 000 000 , on effectue l'isolation en masse de tous ces vecteurs d'expression et la retransformation de 100 ensembles distincts de 10⁸ bactéries avec un rapport de vecteurs à bactéries suffisamment faible pour que, en moyenne chaque ensemble de bactéries soit transformé par seulement environ la moitié du nombre de gène initiaux, c'est-à-dire environ 500 000. Par conséquent,la probabilité pour qu'un quelconque de ces 100 ensembles de bactéries contienne l'ensemble des 5 nouvelles protéines critiques est égal à (1/2)⁵=1/32. Parmi les 100 ensembles initiaux de bactéries, environ 3 contiendront les 5 transformants critiques. Dans chacun de ces ensembles, la quantité totale de gênes nouveaux présente n'est plus que de 500 000 au lieu de un million. Par répétitions successives, dont le nombre total est dans le cas présent de 20, de cette procédure on peut isoler les 5 nouveaux gènes critiques. Après quoi, la mutagénèse et la sélection de cet ensemble de 5 gènes stochastique permet la recherche de fonctions catalytiques améliorées. Dans le cas où il est nécessaire de catalyser une séquence de réactions comprenant un nombre de réactions de l'ordre de 20 et que 20 gènes codant des nouvelles protéines doivent être isolés en parallèle, il suffit d'ajuster la multiplicité des transformations de sorte que chaque ensemble de 10⁸ bactéries reçoivent 80% des 10⁶ gènes stochastiques en utilisant 200 ensembles de ce genre. La probabilité que les 20 nouvelles protéines se trouvent dans un ensemble donné de bactéries est de 0,8²⁰ c'est-à-dire environ 0,015. Par conséquent, 2 parmi les 200 ensembles contiendront les 20 nouveaux gènes nécessaires pour catalyser la formation du composé cible. Le nombre de répétitions de cycles nécessaires pour l'isolation des 20 nouveaux gènes est de l'ordre de 30.

Les principes et les modes opératoires énoncés ci-dessus se généralisent du cas des peptides à de nombreux domaines de la chimie dans lesquels on peut effectuer des réactions en milieu aqueux dans des conditions de pH, température et concentration des solutions permettant les fonctions enzymatiques générales. Dans chaque cas, il est nécessaire de pouvoir disposer d'une méthode d'essai pour déceler la formation du ou des composés cibles désirés. Il faut aussi choisir un nombre suffisamment élevé de "blocs de construction" pour augmenter le nombre de séquences de réaction qui conduisent au composé cible.

L'ensemble concret qui vient d'être donné de la synthèse d'un pentapeptide cible peut être généralisé de la manière suivante :

Le procédé tel qu'il est décrit, engendre, entre autres produits, des peptides et protéines stochastiques. Ces peptides ou protéines peuvent agir, par voie catalytique ou autre, sur d'autres composés. Ils peuvent également constituer les substrats sur lesquels ils agissent. On peut ainsi sélectionner (ou cribler) la capacité qu'ont les peptides ou protéines stochastiques d'interagir entre eux et de ce fait de modifier la conformation, la structure ou la fonction de certains d'entre eux. De même, on peut sélectionner (ou cribler) la capacité de ces peptides et protéines à catalyser entre eux l'hydrolyse, la condensation, le transpeptidation ou d'autres réactions de modification. Par exemple, l'hydrolyse d'une protéine stochastique donnée par un membre au moins de l'ensemble des peptides et protéines stochastiques peut être suivie et mesurée par marquage radioactif de la protéine donnée suivi d'une incubation avec le mélange des protéines stochastiques, en présence d'ions tels que Mg, Ca, Zn, Fe et des composés ATP et GTP. On mesure ensuite l'apparition des fragments radioactifs de la protéine marquée, comme il a été décrit. La ou les protéines stochastiques qui catalysent cette réaction peuvent alors être isolées, ainsi que leur gène producteur, par diminution séquentielle de la librairie de clones transformants, comme décrit.

Une extension du procédé consiste en la sélection d'un ensemble de peptides et polypeptides stochastiques capables de catalyser une suite de réactions menant des constituant de départ (acides aminés et petits peptides) à certains des peptides ou polypeptides de l'ensemble. Il est ainsi envisageable de sélectionner un ensemble capable de catalyser sa propre synthèse : un tel ensemble rétlexivement autocatalytique peut s'établir dans un chemostat où les produits de réaction sont constamment dilués mais où la concentration des produits de départ est maintenues constante. On peut vérifier l'existence d'un ensemble de cette nature par chromatographie sur gel à deux dimensions et par "HPLC" montrant la synthèse d'une distribution stable de peptides et de polypeptides. Les volumes de réaction dépendent du nombre d'espèces moléculaires utilisées et des concentrations nécessaires pour favoriser la formation de liaisons peptidiques par rapport à l'hydrolyse. La distribution des espèces moléculaires d'un ensemble autocatalytique est susceptible de varier ou de dériver par suite de l'émergence d'ensembles autocatalytiques variants. Les peptides et polypeptides qui constituent un ensemble autocatalytique peuvent avoir certains éléments en commun avec le vaste ensemble de départ (constitué des peptides et polypeptides codés selon le procédé) mais peuvent aussi contenir des peptides et polypeptides non codés par l'ensemble des gênes stochastiques codant l'ensemble de départ.

L'ensemble des gènes stochastiques dont les produits sont nécessaire pour établir un tel ensemble autocatalytique peut être isolé comme il a été décrit, par diminutions séquentielles de la libraire de clones transformants. Par ailleurs, un ensemble autocatalytique peut contenir des peptides codés initialement par les gênes stochastiques et formés de manière continue dans l'ensemble autocatalytique. Pour isoler ce sous-ensemble codé de peptides et de protéines on peut utiliser l'ensemble autocatalytique pour obtenir, par immunisation chez l'animal, des sérums polyclonaux reconnaissant un très grand nombre des constituants de l'ensemble autocatalytique.

Ces sérums peuvent ensuite être utilisés pour cribler la librairie de gènes stochastiques pour y trouver les gênes exprimant des protéines capables de se combiner aux anticorps présents dans les sérums.

Cet ensemble de gènes stochastiques exprime un grand nombre des protéines stochastiques codées qui persistent dans l'ensemble autocatalytique. Le reste des constituants codés d'un tel ensemble autocatalytique peut être isolé par diminution séquentielle, comme décrit, de la librairie de gènes stochastiques dont le sous-ensemble détecté par la méthode immunologique a été soustrait.

Les ensembles autocatalytiques de peptides et de protéines obtenue de la manière qui vient d'être décrite sont susceptibles de trouver de nombreuses applications pratiques.

## Revendications

1. Procédé de production d'une pluralité de peptides, polypeptides et/ou protéines ayant l'aptitude de catalyser une séquence de réactions conduisant d'un groupe initial donné de composés chimiques à au moins un composé cible, **caractérisé en ce que** l'on fournit simultanément, au sein d'un même milieu, une pluralité de gènes au moins partiellement constitués de polynucléotides synthétiques stochastiques, de façon à produire une bibliothèque de gènes, on amplifie les gènes, on effectue un criblage et/ou une sélection, de façon à identifier une pluralité de gènes capables d'être exprimés sous forme d'un ensemble composé de peptides, polypeptides et/ou protéines ayant ladite aptitude catalytique, et l'on produit un tel ensemble de peptides, polypeptides et/ou protéines en utilisant l'information génétique ainsi obtenue.

2. Procédé selon la revendication 1 pour la production d'un ensemble constitué d'une pluralité de peptides, polypeptides et/ou proteines réflexivement autocatalytique, **caractérisé par le fait que** ladite aptitude catalytique est l'aptitude de catalyser la synthèse de cet ensemble lui-même à partir d'acides aminés et/ou d'oligopeptides.

3. Procédé de production de peptides, polypeptides ou protéines ayant au moins une propriété donnée, selon lequel on fournit simultanément, au sein d'un même milieu, une pluralité de gènes constitués de polynucléotides synthétiques stochastiques, de façon à produire une bibliothèque de gènes, on amplifie les gênes, on effectue un criblage et/ou une sélection, de façon à identifier au moins un gène capable d'être exprimé sous forme de peptide, polypeptide ou protéine ayant ladite propriété, et l'on produit au moins un peptide, polypeptide ou protéine en utilisant l'information génétique ainsi obtenue, **caractérisé par le fait que** ladite propriété est l'aptitude à modifier sélectivement les propriétés chimiques et/ou biologiques d'un composé donné.

4. Procédé de production d'ARN ayant au moins une propriété donnée, selon lequel on fournit simultanément, au sein d'un même milieu, une pluralité de gènes constitués de polynucléotides synthétiques stochastiques, de façon à produire une bibliothèque de gènes, on amplifie les gènes, on effectue un criblage et/ou une sélection, de façon à identifier au moins un gène capable de produire un ARN ayant ladite propriété, **caractérisé par le fait que** ladite propriété est l'aptitude à se lier à un ligand donné.

5. Procédé de production d'ARN ayant au moins une propriété donnée, selon lequel on fournit simultanément, au sein d'un même milieu, une pluralité de gènes constitués de polynucléotides synthétiques stochastiques, de façon à produire une bibliothèque de gènes, on introduit lesdits gènes dans des cellules-hôtes, de façon à former une bibliothèque de cellules-hôtes transformées correspondant à ladite bibliothèque de gènes, on effectue un criblage et/ou une sélection, de façon à identifier au moins une cellule-hôte contenant des séquences stochastiques d'ARN ayant ladite propriété, **caractérisé par le fait que** ladite propriété est l'aptitude à se lier à un ligand donné.

6. Procédé selon la revendication 3, **caractérisé par le fait que** ladite propriété est l'aptitude à simuler ou modifier au moins une fonction biologique d'au moins un composé biologiquement actif.

7. Procédé selon la revendication 4, **caractérisé par le fait que** ledit composé biologiquement actif est choisi parmi les hormones, les neurotransmetteurs, les facteurs d'adhésion ou de croissance et les régulateurs spécifiques de la réplication et/ou de la transcription de l'ADN et/ou de la traduction de l'ARN.

8. Procédé de production de peptides, polypeptides ou protéines ayant au moins une propriété donnée, selon lequel on fournit simultanément, au sein d'un même milieu, une pluralité de gènes au moins partiellement constitués de polynucléotides synthétiques stochastiques, de façon à produire une bibliothèque de gènes, on amplifie les gènes, on effectue un criblage et/ou une sélection, de façon à identifier au moins un gène capable d'être exprimé sous forme de peptide, polypeptide ou protéine ayant ladite propriété, et l'on produit au moins un peptide, polypeptide ou protéine en utilisant l'information génétique ainsi obtenue, **caractérisé par le fait que** ladite propriété est d'avoir au moins un épitope semblable à l'un des épitopes d'un antigène donné.

9. Procédé de production de peptides, polypeptides ou protéines ayant au moins une propriété donnée, selon lequel on fournit simultanément, au sein d'un même milieu, une pluralité de gènes au moins partiellement constitués de polynucléotides synthétiques stochastiques, de façon à produire une bibliothèque de gènes, on amplifie les gènes, on effectue un criblage et/ou une sélection, de façon à identifier au moins un gène capable d'être exprimé sous forme de peptide, polypeptide ou protéine ayant ladite propriété, et l'on produit au moins un peptide, polypeptide ou protéine en utilisant l'information génétique ainsi obtenu, ce procédé comprenant, en outre, l'introduction desdits gènes dans des cellules-hôtes, de façon à produire une bibliothèque de cellules-hôtes modifiées renfermant ces gènes, le criblage et/ou la sélection étant effectués sur la bibliothèque de cellules-hôtes modifiées ainsi obtenues, **caractérisé par le fait que** ladite propriété est l'aptitude à simuler un épitope d'un antigène donné et que l'on effectue le criblage et/ou la sélection de la souche de cellules-hôtes modifiée produisant au moins un peptide, polypeptide ou protéine ayant cette propriété en préparant des anticorps ayant la propriété de se lier sur cet épitope, et en utilisant ces anticorps, après leur purification, pour identifier les souches contenant ce peptide ou polypeptide, puis en cultivant les souches ainsi identifiées et en séparant et en purifiant le peptide, polypeptide ou protéine produit par ces souches et, finalement, en soumettant ce peptide, ou polypeptide ou cette protéine à un essai in vitro pour vérifier qu'il, ou elle, présente bien ladite propriété.

10. Procédé de production de peptides, polypeptides ou protéines ayant au moins une propriété donnée, selon lequel on fournit simultanément, au sein d'un même milieu, une pluralité de gènes au moins partiellement constitués de polynucléotides synthétiques stochastiques, de façon à produire une bibliothèque de gènes, on amplifie les gènes, on effectue un criblage et/ou une sélection, de façon à identifier au moins un gène capable d'être exprimé sous forme de peptide, polypeptide ou protéine ayant ladite propriété, et l'on produit au moins un peptide, polypeptide ou protéine en utilisant l'information génétique ainsi obtenue, ce procédé comprenant, en outre, le clonage desdits gènes dans ces virus, de façon à produire une bibliothèque de plaques virales modifiées correspondant à cette bibliothèque de gènes, le criblage et/ou la sélection étant effectués sur la bibliothèque de plaques virales modifiées ainsi obtenue, **caractérisé par le fait que** ladite propriété est l'aptitude à simuler un épitope d'un antigène donné.

11. Procédé de production de peptides, polypeptides ou protéines ayant au moins une propriété donnée, selon lequel on fournit simultanément, au sein d'un même milieu, une pluralité de gènes au moins partiellement constitués de polynucléotides synthétiques stochastiques, de façon à produire une bibliothèque de gènes, on amplifie les gènes, on effectue un criblage et/ou une sélection, de façon à identifier au moins un gène capable d'être exprimé sous forme de peptide, polypeptide ou protéine ayant ladite propriété, et l'on produit au moins un peptide, polypeptide ou protéine en utilisant l'information génétique ainsi obtenu, ce procédé comprenant, en outre, l'introduction desdits gènes dans des cellules-hôtes, de façon à produire une bibliothèque de cellules-hôtes modifiées renfermant ces gènes, le criblage et/ou la sélection étant effectués sur la bibliothèque de cellules-hôtes modifiées ainsi obtenue, **caractérisé par le fait que** ladite propriété est l'aptitude à simuler un site de liaison d'une molécule donnée pour un récepteur donné et que l'on effectue le criblage et/ou la sélection de la souche de cellules-hôtes modifiée produisant au moins un peptide, polypeptide ou protéine ayant cette propriété en obtenant une certaine quantité du récepteur et en utilisant cette quantité de récepteur pour identifier les souches contenant ce peptide ou polypeptide qui se lient avec ledit récepteur, puis en cultivant les souches ainsi identifiées et en séparant et en purifiant le peptide, polypeptide ou protéine produit par ces souches et, finalement, en soumettant ce peptide, ou polypeptide ou cette protéine à un essai in vitro pour vérifier qu'il, ou elle, présente bien ladite propriété.

12. Utilisation de peptides, polypeptides ou protéines obtenus par le procédé selon la revendication 6 ou la revendication 7, comme substance active pour la préparation d'un médicament ayant une action pharmacologique et/ou chimiothérapeutique.

13. Utilisation de peptides, polypeptides ou protéines, obtenus par le procédé selon la revendication 6, pour diminuer, in vitro, la concentration en anticorps libres spécifiques contre ledit antigène par ligation entre ces peptides, polypeptides ou protéines et ces anticorps.

14. Utilisation de peptides, polypeptides ou protéines, selon la revendication 8 ou la revendication 9, comme substance active pour la préparation d'un agent suppresseur d'hypersensibilité immunitaire.

15. Utilisation de peptides, polypeptides ou protéines, obtenus par le procédé selon la revendication 8 ou la revendication 9, comme substance active pour la préparation d'un agent de création d'une tolérance à l'égard dudit antigène.

16. Procédé selon la revendication 8, **caractérisé par le fait que** l'antigène est l'EGF.

17. Utilisation de peptides, polypeptides ou protéines obtenus par le procédé selon la revendication 16, comme substance active pour la préparation d'un médicament pour le traitement chimiothérapeutique des épithéliomes.

18. Application du procédé selon la revendication 8 ou la revendication 9 pour la préparation d'un vaccin, **caractérisée par le fait que** ladite propriété est l'aptitude à simuler un épitope d'une autre molécule.

19. Application du procédé selon la revendication 8 pour la préparation d'un vaccin, **caractérisée par le fait que** l'on obtient des anticorps contre un agent pathogène et on les utilise pour identifier au moins un gène capable d'être exprimé sous forme de peptide, polypeptide ou protéine ayant au moins un épitope semblable à l'un des épitopes de l'agent pathogène, on utilise l'information génétique ainsi obtenue pour produire au moins un tel peptide ou polypeptide ou une telle protéine que l'on utilise pour la production d'un vaccin contre l'agent pathogène.

20. Application du procédé selon la revendication 9 pour la préparation d'un vaccin, **caractérisée par le fait que** l'on isole des anticorps contre un agent pathogène et on les utilise pour identifier les clones produisant au moins un peptide ou polypeptide, ou au moins une protéine ayant au moins un épitope semblable à l'un des épitopes de l'agent pathogène, on cultive les souches de cellules-hôtes modifiées correspondant à ces clones, de façon à produire ledit peptide ou polypeptide ou ladite protéine, on isole et purifie ce peptide ou polypeptide ou cette protéine, à partir des cultures de ces souches de cellules, et on l'utilise pour la production d'un vaccin contre l'agent pathogène.

21. Application selon la revendication 19 ou la revendication 20 pour la préparation d'un vaccin anti-HVB, **caractérisée par le fait que** l'on extrait et l'on purifie au moins une protéine de capside de virus HVB, on injecte cette protéine dans l'organisme d'un animal capable de former des anticorps contre cette protéine, on recueille et on purifie les anticorps ainsi formés, on utilise ces anticorps pour identifier les clones produisant au moins un peptide ou polypeptide ou au moins une protéine ayant au moins un épitope semblable à l'un des épitopes du virus HVB, on cultive les souches de cellules-hôtes modifiées correspondant à ces clones, de façon à produire ledit peptide ou polypeptide ou ladite protéine, on isole et purifie ce peptide ou polypeptide ou cette protéine à partir des cultures de ces souches de cellules, et on l'utilise pour la production d'un vaccin anti-HVB.

22. Procédé de production de peptides, polypeptides ou protéines ayant au moins une propriété donnée, selon lequel on fournit simultanément, au sein d'un même milieu, une pluralité de gènes au moins partiellement constitués de polynucléotides synthétiques stochastiques, de façon à produire une biliothèque de gènes, on amplifie les gènes, on effectue un criblage et/ou une sélection, de façon à identifier au moins un gène capable d'être exprimé sous forme de peptide, polypeptide ou protéine ayant ladite propriété, et l'on produit au moins un peptide, polypeptide ou protéine en utilisant l'information génétique ainsi obtenue, **caractérisé par le fait que** ladite propriété est l'aptitude à se lier à un composé régulateur de l'activité de transcription ou de réplication de l'ADN, l'activité de traduction de l'ARN ou d'autres activités moléculaires.

23. Utilisation d'un peptide ou polypeptide, ou d'une protéine, obtenu par un procédé selon lequel on fournit simultanément, au sein d'un même milieu, une pluralité de gènes au moins partiellement constitués de polynucléotides synthétiques stochastiques, de façon à produire une bibliothèque de gènes, on amplifie les gènes, on effectue un criblage et/ou une sélection, de façon à identifier au moins un gène capable d'être exprimé sous forme de peptide, polypeptide ou protéine ayant l'aptitude à se lier à un ligand donné, et l'on produit au moins un peptide, polypeptide ou protéine en utilisant l'information génétique ainsi obtenue pour la détection et/ou la titration d'un ligand.

24. Procédé selon la revendication 22, **caractérisé par le fait que** ledit composé est une protéine régulatrice de l'activité de transcription ou de réplication de l'ADN.

25. Procédé selon la revendication 22, **caractérisé par le fait que** ledit composé est choisi parmi les séquences d'ADN et d'ARN.

26. Utilisation d'une séquence d'ADN obtenue par un procédé selon lequel on fournit simultanément, au sein d'un même milieu, une pluralité de gènes au moins partiellement constitués de polynucléotides synthétiques stochastiques, de façon à produire une bibliothèque de gène, on amplifie les gènes, on effectue un criblage et/ou une sélection, de façon à identifier au moins un gène contenant une séquence d'ADN ayant l'aptitude à se lier à un composé régulateur de l'activité de transcription ou de réplication de l'ADN, et on produit une telle séquence d'ADN en utilisant l'information génétique ainsi obtenue, comme séquence cis-régulatrice de la réplication ou de la transcription d'une séquence d'ADN voisine.

27. Utilisation d'une protéine, obtenue par le procédé selon la revendication 22, pour modifier les propriétés de transcription ou de réplication ou de stabilité d'une séquence d'ADN, dans une cellule contenant cette séquence d'ADN et exprimant cette protéine.

28. Procédé de production d'un ensemble de peptides, polypeptides ou protéines capable de catalyser une séquence de réactions chimiques conduisant à la formation d'au moins un composé désiré, **caractérisé en ce qu'**il comprend les opérations suivantes:
a) production d'une bibliothèque de séquences de polynucléotide au moins partiellement composées de polynucléotides synthétiques stochastiques;
b) introduction des séquences de polynuclétode, ainsi obtenues, dans des cellules-hôtes;
c) culture simultanée des cellules-hôtes transformées renfermant lesdites séquences de polynucléotides, de manière à cloner ces séquences de polynucléotides et provoquer la production d'un premier ensemble de peptides, polypeptides ou protéines exprimés par au moins certaines de ces séquences;
d) combinaison de ce premier ensemble de peptides, polypeptides ou protéines avec une collection de précurseurs pour ledit composé désiré, dans des conditions favorables à ladite séquence de réactions chimiques;
e) détection de la formation éventuelle dudit composé désiré et, dans le cas où cette formation est effectivement détectée, séparation du premier ensemble de peptides, polypeptides ou protéines en une pluralité de sons-ensembles;
f) combinaison de chacun de ces sous-ensembles avec ladite collection de précurseurs, dans des conditions favorables à ladite séquence de réactions chimiques;
g) détection, dans chaque sous-ensemble, de la formation éventuelle dudit composé désiré et séparation de chaque ensemble, dans lequel la formation de ce composé est effectivement détectée, en une nouvelle pluralité de sous-ensembles; et
h) répétition des opérations f) et g) jusqu'à identification de l'ensemble de peptides, polypeptides ou protéines qui catalyse la séquence de réactions chimiques conduisant à la formation dudit composé désiré.

29. Procédé de production d'un ensemble de peptides, polypeptides ou protéines capable de catalyser une séquence de réactions chimiques conduisant à la formation d'au moins un composé désiré, **caractérisé en ce qu'**il comprend les opérations suivantes:
a) production d'une bibliothèque de séquences de polynucléotide au moins partiellement composées de polynucléotides synthétiques stochastiques;
b) introduction des séquences de polynuclétode, ainsi obtenues, dans des cellules-hôtes;
c) culture simultanée des cellules-hôtes transformées renfermant lesdites séquences de polynucléotides, de manière à produire un premier ensemble de cellules-hôtes et à cloner les séquences de polynucléotides, de façon à provoquer la production d'un premier ensemble de peptides, polypeptides ou protéines exprimés par au moins certaines de ces séquences;
d) combinaison des peptides, polypeptides ou protéines produits par ce premier ensemble de cellules-hôtes avec une collection de précurseurs pour ledit composé désiré, dans des conditions favorables à ladite séquence de réactions chimiques;
e) détection de la formation éventuelle dudit composé désiré et, dans le cas où cette formation est effectivement détectée, séparation du premier ensemble de cellules-hôtes en une pluralité de sous-ensembles;
f) combinaison des peptides, polypeptides ou protéines produits par chacun de ces sous-ensembles de cellules-hôtes avec ladite collection de précurseurs, dans des conditions favorables à ladite séquence de réactions chimiques;
g) détection, dans chaque sous-ensemble, de la formation éventuelle dudit composé désiré avec les peptides, polypeptides ou protéines produits par chacun d'eux, et séparation de chaque ensemble, dans lequel la formation de ce composé est effectivement détectée, en une nouvelle pluralité de sous-ensembles; et
h) répétition des opérations f) et g) jusqu'à identification de l'ensemble de peptides, polypeptides ou protéines qui catalyse la séquence de réactions chimiques conduisant à la formation dudit composé désiré.

30. Procédé de production d'un ensemble de peptides, polypeptides ou protéines capable de catalyser une séquence de réactions chimiques conduisant à la formation d'au moins un composé désiré, **caractérisé en ce qu'**il comprend les opérations suivantes:
a) opération selon laquelle on fournit, dans un premier ensemble, un grand nombre de peptides, polypeptides ou protéines différents, dont au moins une partie ont l'aptitude de catalyser une séquence de réactions chimiques conduisant à la formation dudit composé désiré;
b) combinaison de ce premier ensemble de peptides, polypeptides ou protéines avec une collection de précurseurs pour ledit composé désiré, dans des conditions favorables à ladite séquence de réactions chimiques;
c) détection de la formation éventuelle dudit composé désiré et, dans le cas où cette formation est effectivement détectée, séparation du premier ensemble de peptides, polypeptides ou protéines en une pluralité de sous-ensembles;
d) combinaison de chacun de ces sous-ensembles avec ladite collection de précurseurs, dans des conditions favorables à ladite séquence de réactions chimiques;
e) détection, dans chaque sous-ensemble, de la formation éventuelle dudit composé désiré et séparation de chaque ensemble, dans lequel la formation de ce composé est effectivement détectée, en une nouvelle pluralité de sous-ensembles; et
f) répétition des opérations d) et e) jusqu'à identification d'un ensemble de peptides, polypeptides ou protéines qui catalyse la séquence de réactions chimiques conduisant à la formation dudit composé désiré.

31. Utilisation d'un ensemble de peptides, polypeptides ou protéines ayant une activité enzymatique, obtenu par le procédé selon la revendication 29 ou la revendication 30, pour la production d'un composé chimique ayant une propriété désirée, **caractérisée en ce qu'**elle comprend les opérations suivantes:
a) mise en réaction dudit ensemble, dans lequel les différents peptides, polypeptides ou protéines correspondent à une pluralité d'activités catalytiques différentes, avec un groupe constitué d'une pluralité de substrats différents, de façon à produire au moins un composé chimique différant des peptides, polypeptides ou protéines de départ présents dans le mélange réactionnel;
b) criblage et/ou sélection du mélange réactionnel en vue de l'identification de la présence d'un composé chimique ayant ladite propriété désirée, et
c) la séparation dudit composé chimique du mélange réactionnel.

32. Procédé de production d'un composé, en utilisant un ensemble de peptides, polypeptides ou protéines capable de catalyser une séquence de réactions chimiques conduisant à la formation de ce composé, **caractérisé en ce qu'**il comprend les opérations suivantes:
a) production d'une bibliothèque de séquences de polynucléotide au moins partiellement composées de polynucléotides synthétiques stochastiques;
b) introduction des séquences de polynuclétode, ainsi obtenues, dans des cellules-hôtes;
c) culture simultanée des cellules-hôtes transformées renfermant lesdites séquences de polynucléotides, de manière à cloner les séquences de polynucléotides stochastiques et provoquer la production d'un premier ensemble de peptides, polypeptides ou protéines exprimés par au moins certaines de ces séquences de polynucléotides;
d) combinaison de ce premier ensemble de peptides, polypeptides ou protéines avec une collection de précurseurs pour ledit composé désiré, dans des conditions favorables à ladite séquence de réactions chimiques;
e) détection de la formation éventuelle dudit composé désiré et, dans le cas où cette formation est effectivement détectée, séparation du premier ensemble de peptides, polypeptides ou protéines en une pluralité de sous-ensembles;
f) combinaison de chacun de ces sous-ensembles avec ladite collection de précurseurs, dans des conditions favorables à ladite séquence de réactions chimiques;
g) détection, dans chaque sous-ensemble, de la formation éventuelle dudit composé désiré et séparation de chaque ensemble, dans lequel la formation de ce composé est effectivement détectée, en une nouvelle pluralité de sous-ensembles;
h) répétition des opérations f) et g) jusqu'à identification de l'ensemble de peptides, polypeptides ou protéines qui catalyse la séquence de réactions chimiques conduisant à la formation dudit composé désiré; et
i) utilisation de l'ensemble de peptides, polypeptides ou protéines, ainsi identifié, pour produire ledit composé désiré à partir d'une collection de précurseurs pour ce composé.

33. Procédé de production d'un composé, en utilisant un ensemble de peptides, polypeptides ou protéines capable de catalyser une séquence de réactions chimiques conduisant à la formation de ce composé, **caractérisé en ce qu'**il comprend les opérations suivantes:
a) production d'une bibliothèque de séquences de polynucléotide au moins partiellement composées de polynucléotides synthétiques stochastiques;
b) introduction des séquences de polynuclétode, ainsi obtenues, dans des cellules-hôtes;
c) culture simultanée des cellules-hôtes transformées renfermant lesdites séquences de polynucléotides, de manière à obtenir un premier ensemble de cellules-hôtes cultivées et à cloner les séquences de polynucléotides et provoquer la production d'un premier ensemble de peptides, polypeptides ou protéines exprimés par au moins certaines de ces séquences de polynucléotides;
d) combinaison de ce premier ensemble de peptides, polypeptides ou protéines produits par le premier ensemble de cellules-hôtes cultivées avec une collection de précurseurs pour ledit composé désiré, dans des conditions favorables à ladite séquence de réactions chimiques;
e) détection de la formation éventuelle dudit composé désiré et, dans le cas où cette formation est effectivement détectée, séparation du premier ensemble de cellules-hôtes cultivées en une pluralité de sous-ensembles;
f) combinaison des peptides, polypeptides ou protéines produits par chacun de ces sous-ensembles avec ladite collection de précurseurs, dans des conditions favorables à ladite séquence de réactions chimiques;
g) détection, dans chaque sous-ensemble, de la formation éventuelle dudit composé désiré avec les peptides, polypeptides ou protéines produits par chacun de ces sous-ensembles et séparation de chaque ensemble, dans lequel la formation de ce composé est effectivement détectée, en une nouvelle pluralité de sous-ensembles;
h) répétition des opérations f) et g) jusqu'à identification de l'ensemble de peptides, polypeptides ou protéines qui catalyse la séquence de réactions chimiques conduisant à la formation dudit composé désiré; et
i) utilisation de l'ensemble de peptides, polypeptides ou protéines, ainsi identifié, pour produire ledit composé désiré à partir d'une collection de précurseurs pour ce composé.

34. Procédé de production d'un composé, en utilisant un ensemble de peptides, polypeptides ou protéines capable de catalyser une séquence de réactions chimiques conduisant à la formation de ce composé, **caractérisé en ce qu'**il comprend les opérations suivantes:
a) opération selon laquelle on fournit, dans un premier ensemble, un grand nombre de peptides, polypeptides ou protéines différents, dont au moins une partie ont l'aptitude de catalyser une séquence de réactions chimiques conduisant à la formation dudit composé désiré;
b) combinaison de ce premier ensemble de peptides, polypeptides ou protéines avec une collection de précurseurs pour ledit composé désiré, dans des conditions favorables à ladite séquence de réactions chimiques;
c) détection de la formation éventuelle dudit composé désiré et, dans le cas où cette formation est effectivement détectée, séparation du premier ensemble de peptides, polypeptides ou protéines en une pluralité de sous-ensembles;
d) combinaison de chacun de ces sous-ensembles avec ladite collection de précurseurs, dans des conditions favorables à ladite séquence de réactions chimiques;
e) détection, dans chaque sous-ensemble, de la formation éventuelle dudit composé désiré et séparation de chaque ensemble, dans lequel la formation de ce composé est effectivement détectée, en une nouvelle pluralité de sous-ensembles;
f) répétition des opérations d) et e) jusqu'à identification de l'ensemble de peptides, polypeptides ou protéines qui catalyse la séquence de réactions chimiques conduisant à la formation dudit composé désiré; et
g) utilisation de l'ensemble de peptides, polypeptides ou protéines, ainsi identifié, pour produire ledit composé désiré à partir d'une collection de précurseurs pour ce composé.

35. Procédé de production d'une séquence d'ARN ayant au moins une propriété donnée, selon lequel on produit une bibliothèque de gènes, on amplifie et on traduit ces gènes de façon à produire des peptides ou polypeptides ou des protéines exprimés par au moins l'un des gènes de cette bibliothèque, l'on effectue un criblage et/ou une sélection de façon à identifier au moins une séquence d'ARN ayant ladite propriété, et l'on produit une telle séquence d'ARN en utilisant l'information génétique ainsi obtenue, **caractérisé en ce que** les gènes sont, au moins partiellement, composés de polynucléotides synthétiques stochastiques.

36. Bibliothèque de gènes, **caractérisée en ce que** chacun des gènes est, au moins partiellement, composé de polynucléotides synthétiques stochastiques, en tant que produit intermédiaire dans le procédé selon l'une des revendications 1, 3 et 8.

37. Bibliothèque de cellules-hôtes transformées, **caractérisée en ce que** chacune des cellules-hôtes transformée contient au moins un gène au moins partiellement composé de polynucléotides synthétiques stochastiques, en tant que produit intermédiaire dans le procédé selon la revendication 9.

38. Bibliothèque de gènes, **caractérisée en ce que** chacun des gènes est, au moins partiellement, composé de polynucléotides synthétiques stochastiques, en tant que produit intermédiaire dans un procédé de production de peptides, polypeptides ou protéines ayant au moins une propriété donnée, selon lequel on fournit simultanément, au sein d'un même milieu, une pluralité de gènes au moins partiellement composés de polynucléotides synthétiques stochastiques, de façon à produire une bibliothèque de gènes, on amplifie les gènes, on effectue un criblage et/ou une sélection, de façon à identifier au moins un gène capable d'être exprimé sous forme de peptide, polypeptide ou protéine ayant ladite propriété, et l'on produit au moins un peptide, polypeptide ou protéine en utilisant l'information génétique ainsi obtenue.

39. Bibliothèque de gènes, **caractérisée en ce que** chacun des gènes est, au moins partiellement, composé de polynucléotides synthétiques stochastiques, en tant que produit intermédiaire dans un procédé de production d'ADN ou d'ARN ayant au moins une propriété donnée, selon lequel on fournit simultanément, au sein d'un même milieu, une pluralité de gènes au moins partiellement composés de polynucléotides synthétiques stochastiques, de façon à produire une bibliothèque de gènes, on amplifie les gènes, on effectue un criblage et/ou une sélection, de façon à identifier au moins un gène capable d'être exprimé sous forme d'ADN ou d'ARN ayant ladite propriété, et l'on produit au moins un ADN ou ARN en utilisant l'information génétique ainsi obtenue.

40. Bibliothèque de cellules-hôtes transformées, **caractérisée en ce que** chacune des cellules-hôtes transformée contient au moins un gène au moins partiellement composé de polynucléotides synthétiques stochastiques, en tant que produit intermédiaire dans un procédé de production de peptides, polypeptides ou protéines ayant au moins une propriété donnée, selon lequel on fournit simultanément, au sein d'un même milieu, une pluralité de gènes au moins partiellement composés de polynucléotides synthétiques stochastiques, de façon à produire une bibliothèque de gènes, on amplifie les gènes, on effectue un criblage et/ou une sélection, de façon à identifier au moins un gène capable d'être exprimé sous forme de peptide, polypeptide ou protéine ayant ladite propriété, et l'on produit au moins un peptide, polypeptide ou protéine en utilisant l'information génétique ainsi obtenue, ce procédé comprenant, en outre, l'introduction desdits gènes dans des cellules-hôtes, de façon à produire une bibliothèque de cellules-hôtes modifiées renfermant ces gènes, le criblage et/ou la sélection étant effectués sur la bibliothèque de cellules-hôtes modifiées ainsi obtenues.

41. Bibliothèque de cellules-hôtes transformées, **caractérisée en ce que** chacune des cellules-hôtes transformée contient au moins un gène au moins partiellement composé de polynucléotides synthétiques stochastiques, en tant que produit intermédiaire dans un procédé de production d'ADN ou d'ARN ayant au moins une propriété donnée, selon lequel on fournit simultanément, au sein d'un même milieu, une pluralité de gènes au moins partiellement composés de polynucléotides synthétiques stochastiques, de façon à produire une bibliothèque de gènes, on amplifie les gènes, on effectue un criblage et/ou une sélection, de façon à identifier au moins un gène capable d'être exprimé sous forme de peptide, polypeptide ou protéine ayant ladite propriété, et l'on produit au moins un ADN ou ARN en utilisant l'information génétique ainsi obtenue, ce procédé comprenant, en outre, l'introduction desdits gènes dans des cellules-hôtes, de façon à produire une bibliothèque de cellules-hôtes modifiées renfermant ces gènes, le criblage et/ou la sélection étant effectués sur la bibliothèque de cellules-hôtes modifiées ainsi obtenues.
